# EUROPÄISCHE PATENTSCHRIFT

(11) **EP 3 685 419 B1**
(45) Veröffentlichungstag und Bekanntmachung des Hinweises auf die Patenterteilung: **26.02.2025**
(21) Anmeldenummer: 18778798.1
(22) Anmeldetag: 21.09.2018
(51) Int. Cl.: A61B 6/40, H01J 35/06, H01J 35/24, A61B 6/00

(54) **C-BOGEN-RÖNTGENGERÄT**
C-ARM X-RAY APPARATUS
APPAREIL DE RADIOGRAPHIE À BRAS EN C

(30) Priorität: 21.09.2017 DE 102017008921
(43) Veröffentlichungstag der Anmeldung: 29.07.2020
(73) Patentinhaber: Esspen GmbH, 91052 Erlangen (DE)
(72) Erfinder: ZAHRA, Mohammadi, 91052 Erlangen (DE)
(74) Vertreter: Meyer, Rudolf
(86) Internationale Anmeldenummer: PCT/EP2018/025240
(87) Internationale Veröffentlichungsnummer: WO 2019/057339

(56) Entgegenhaltungen:
- WO-A1-2009/115982
- WO-A1-2009/115982
- DE-A1- 102010 028 438
- DE-A1- 102010 028 438
- DE-A1- 102010 028 438
- US-A1- 2010 172 468
- US-A1- 2010 172 468
- QIAN XIN ET AL: "Design and characterization of a spatially distributed multibeam field emission x-ray source for stationary digital breast tomosynthesis", MEDICAL PHYSICS, AIP, MELVILLE, NY, US, vol. 36, no. 10, 4 September 2009 (2009-09-04), pages 4389 - 4399, XP012129710, ISSN: 0094-2405, DOI: 10.1118/1.3213520
- QIAN XIN ET AL: "Design and characterization of a spatially distributed multibeam field emission x-ray source for stationary digital breast tomosynthesis", MEDICAL PHYSICS, AIP, MELVILLE, NY, US, vol. 36, no. 10, 4 September 2009 (2009-09-04), pages 4389 - 4399, XP012129710, ISSN: 0094-2405, DOI: 10.1118/1.3213520
- QIAN XIN ET AL: "Design and characterization of a spatially distributed multibeam field emission x-ray source for stationary digital breast tomosynthesis", MEDICAL PHYSICS, AIP, MELVILLE, NY, US, vol. 36, no. 10, 4 September 2009 (2009-09-04), pages 4389 - 4399, XP012129710, ISSN: 0094-2405, DOI: 10.1118/1.3213520

## Beschreibung

Die Erfindung betrifft ein C-Bogen-Röntgengerät, das heißt ein Röntgengerät, welches einen C-förmigen Bogen aufweist, an welchem ein Röntgenstrahler sowie ein zugehöriger Röntgendetektor gehalten sind. Weiter betrifft die Erfindung ein Verfahren zum Betrieb eines C-Bogen-Röntgengerätes.

Verschiedene C-Bogen-Röntgengeräte sind zum Beispiel aus den Dokumenten DE 10 2009 033 607 A1, DE 10 2008 059 455 A1, DE10 2010 028438 und US 8, 559,591 B2 bekannt. Im letztgenannten Fall umfasst eine Röntgenquelle Feldemissionskathoden auf der Basis von Kohlenstoff-Nanoröhren (CNT-Kathode; carbon nano tube). Ein Röntgengerät mit CNT-Kathoden ist auch aus WO2009115982 bekannt.

Ein mobiles C-Bogen-Röntgengerät ist zum Beispiel in der DE 10 2011 006 505 A1 offenbart.

US8118488 offenbart ein Tomographiegerät, bei dem ein Röntgenstrahler und ein Röntgendetektor innerhalb eines ringförmigen Gehäuses bewegt werden können, das von einem C-Bogen gehalten wird.

C-Bogen-Röntgengeräte weisen Strahler-Detektor-Anordnungen auf, welche an einem im Wesentlichen C-förmigen Träger, das heißt C-Bogen, befestigt sind. In Kliniken werden C-Bogen-Röntgengeräte beispielsweise zur intraoperativen Bildgebung verwendet.

Der Erfindung liegt die Aufgabe zugrunde, ein C-Bogen-Röntgengerät mit gegenüber dem Stand der Technik erweiterten Möglichkeiten der Bildgebung anzugeben.

Diese Aufgabe wird erfindungsgemäß gelöst durch ein C-Bogen-Röntgengerät mit den Merkmalen des Anspruchs 1. Der Gegenstand des Anspruchs 1 besteht in einem C-Bogen-Röntgengerät, mit einem Röntgenstrahler und einem Röntgendetektor, welche an einem in einer Bezugsebene liegenden C-Bogen gehalten sind, wobei der Röntgenstrahler Nanostäbchen als Elektronenemitter umfasst und eine langgestreckte, schlauchförmige Struktur beschreibt, dadurch gekennzeichnet, dass der Röntgenstrahler eine gekrümmte, langgestreckte Struktur aufweist, wobei eine mittig an den Röntgenstrahler gelegte Tangente eine Flächennormale der Bezugsebene darstellt und die gekrümmte Form des Röntgenstrahlers eine orthogonal zur Bezugsebene ausgerichtete Ebene aufspannt, und wobei die gesamte Erstreckung des Röntgenstrahlers mindestens das Vierfache des quer zur langgestreckten Struktur gemessenen Durchmessers des Querschnitts des Röntgenstrahlers beträgt. Damit unterscheidet sich das erfindungsgemäße C-Bogen-Röntgengerät grundsätzlich von bekannten C-Bogen-Geräten, wie beispielsweise in der genannten US 8,559,591 B2 offenbart, bei welchen eine Anordnung aus mehreren Röntgenquellen in der Bezugsebene liegt. Die Ausrichtung des Röntgenstrahlers quer zur Bezugsebene ermöglicht in weitem Umfang die Generierung von Schnittbildern eines Untersuchungsobjektes ohne jede Verstellung des C-Bogens. Dies begünstigt sowohl einen zeitsparenden Betrieb des für die Tomographie geeigneten C-Bogen-Röntgengerätes als auch eine hohe Qualität erzeugter Bilddaten.

In einfacher Gestaltung, die nicht unter den Schutzbereich des Anspruchs fällt, weist der Röntgenstrahler des C-Bogen-Röntgengerätes eine gerade, langgestreckte, im Wesentlichen zylindrische Form auf. Die Mittelachse des Röntgenstrahlers, insbesondere Zylinderlängsachse, ist hierbei mit einer Flächennormalen der Bezugsebene identisch.

In einer angewandelten Bauform weist der Röntgenstrahler eine gekrümmte Form auf, wobei eine mittig an den Röntgenstrahler gelegte Tangente eine Flächennormale der Bezugsebene darstellt. Insgesamt spannt der gekrümmte Röntgenstrahler eine Ebene auf, die orthogonal zur Bezugsebene ausgerichtet ist. Die Krümmung des Röntgenstrahlers kann beispielsweise in Form eines Kreisbogens oder in U-Form gestaltet sein.

Fügt man gedanklich die beiden Enden eines U-förmig gebogenen Röntgenstrahlers zusammen, so gelangt man zu einer Ringform des Röntgenstrahlers. In einer solchen Ausgestaltung stellen zwei zueinander parallele, an den Röntgenstrahler gelegte Tangenten jeweils eine Flächennormale der durch den C-Bogen definierten Bezugsebene dar. Der ringförmige Röntgenstrahler kann entweder als offener oder als geschlossener Ring gestaltet sein. Im Fall eines geschlossenen Rings, insbesondere einer Kreisform, gibt die Länge einer geschlossenen, mittig durch den gesamten Ring verlaufenden Linie, insbesondere eines Kreises, dessen Durchmesser dem Mittelwert zwischen dem Innendurchmesser und dem Außendurchmesser des insgesamt ringförmigen Röntgenstrahlers entspricht, die gesamte Erstreckung des Röntgenstrahlers in Längsrichtung der langgestreckten Struktur an.

Statt einer Ringform kann der Strahler zum Beispiel auch eine Polygonform aufweisen. Hierbei ist die Erstreckung des Röntgenstrahlers in Längsrichtung der langgestreckten Struktur definiert als Summe der Längen der einzelnen Seiten des Polygons, wobei die Seitenlängen jeweils in der Mitte des Querschnitts des Röntgenstrahlers zu messen sind. Räumlich stellt die polygonförmige Röntgenröhre eine Abwandlung eines Torus dar. Eine Ringform, das heißt Torusform, oder eine Polygonform kann auch durch eine Anordnung aus mehreren, jeweils Nanostäbchen enthaltenden Röntgenstrahlern gebildet sein.

Unabhängig davon, ob der Röntgenstrahler vollständig gerade langgestreckt oder gekrümmt langgestreckt, sei es in einem offenen Bogen oder in einem geschlossenen Ring, ist, beträgt die gesamte, in jedem Abschnitt der langgestreckten Struktur in deren Längsrichtung gemessene Erstreckung des Röntgenstrahlers mindestens das Vierfache des quer zur langgestreckten Struktur gemessenen maximalen Durchmessers des Querschnitts des Röntgenstrahlers.

Die zur Emission von Elektronen vorgesehen Kathoden des Röntgenstrahlers umfassen vorzugsweise Kohlenstoffnanoröhren als Nanostäbchen. Die sehr hohe elektrische und thermische Leitfähigkeit von Kohlenstoffnanoröhren ermöglicht eine hohe Stromtragfähigkeit ohne nennenswerte Hitzeentwicklung der einzelnen Kohlenstoffnanoröhren selbst. Kohlenstoffnanoröhren weisen einen niedrigen Feldstärke-Schwellenwert von weniger als 2 V /m für die Feldemission von Elektronen auf. Der Feldstärke-Schwellenwert bei Kathoden zur Emission von Elektronen, welche Kohlenstoffnanoröhren aufweisen, ist noch weiter absenkbar, indem die Kohlenstoffnanoröhren in senkrechter Vorzugsrichtung auf der Kathodenoberfläche angeordnet sind. Da einwandige Kohlenstoffnanoröhren Halbleiter und mehrwandige Kohlenstoffnanoröhren metallische Leiter darstellen, sind mehrwandige Kohlenstoffnanoröhren für Anwendungen als Elektronenemitter auf den Kathoden des Röntgenstrahlers besonders geeignet.

Außer Kohlenstoffnanoröhren sich auch Nanostäbchen anderer Art, allgemein auch als Nanosticks bezeichnet, für die Emission von Elektronen innerhalb des Röntgenstrahlers geeignet. In bevorzugter Ausgestaltung sind aus solchen Nanosticks Feldemissionskathoden als Kathoden der Röntgenröhre gebildet.

Die Nanosticks der Kathode sind vorzugsweise aus einem Material beschaffen, welches bezüglich des quantenmechanischen Feldemissionseffektes eine möglichst niedrige Elektronenaustrittsarbeit zur Feldemission von Elektronen aufweist. Die Nanosticks weisen hierbei eine in sich einheitliche oder uneinheitliche Zusammensetzung auf und sind entweder als Hohlkörper, das heißt Röhren, oder massiv ausgebildet. Die Kathoden können hierbei Nanosticks gleicher Art oder einen Mischung verschiedener Arten von Nanosticks aufweisen, wobei sich der Begriff "Art der Nanosticks" auf deren Stoffzusammensetzung und Stoffmodifikation bezieht.

Geeignete Materialien in reiner oder dotierter Form für die Feldemission von Elektronen sind neben ein- oder mehrwandige Kohlenstoffnanoröhren auch ein- oder mehrwandige Hetero-Stickstoff-Kohlenstoffnanoröhren, Boride der seltenen Erden, insbesondere Lanthanhexaborid und Cerhexaborid, Metalloxide, insbesondere TiO₂, MnO, ZnO und Al₂O₃, Metallsulfide, insbesondere Molybdänsulfid, Nitride, insbesondere Bornitrid, Aluminiumnitrid, Kohlenstoffnitrid, Galliumnitrid, Carbide, insbesondere Siliciumcarbid, Silicium. Als Ausgangsprodukte zur Herstellung der Nanosticks, welche beim Betrieb der Kathoden Elektronen emittieren, sind auch stabförmige, optional hohle, Elemente aus polymeren Materialien geeignet. Die Nanosticks der Kathoden sind optional aus Ausgangsprodukten, welche lediglich partiell, insbesondere in Form einer Beschichtung, Polymermaterialen aufweisen, gefertigt.

In einer besonders bevorzugten Ausbildung weisen die Kathoden auf der Oberfläche Nanosticks in einer vertikalen Vorzugsrichtung, das heißt in Richtung zu der Anode des Röntgenstrahlers, auf. Beim Betrieb des Röntgenemitters und bei hinreichendem Abstand untereinander sind an den Spitzen der Nanosticks sehr starke elektrische Felder erzeugbar, wodurch die Emission von Elektronen wesentlich vereinfacht ist.

In einer möglichen Ausführungsform des C-Bogen-Röntgengerätes ist in der Vakuumröhre des Röntgenstrahlers mehr als eine Sorte von Kathoden angeordnet, wobei sich der Begriff "Sorte" sowohl auf die Geometrie als auch auf sonstige Eigenschaften der Kathoden, beispielsweise auf die Werkstoffe, beziehen kann. Kathoden gleicher und unterschiedlicher Sorte sind grundsätzlich in beliebiger Weise sequentiell elektrisch ansteuerbar. Neben den Kathoden selbst können auch Unterschiede hinsichtlich der Fokussierung gegeben sein. Zusammen mit Eigenschaften wie der Flächengeometrie der einzelnen Kathoden sind damit unterschiedliche Elektronenstrahlenbündel und letztlich unterschiedliche Röntgenstrahlenbündel erzeugbar.

Die Nanostäbchen der Kathode weisen zum Beispiel eine Länge von weniger als 20 µm und einen Durchmesser von weniger als 10 nm auf, wobei eine auf die Fläche der Kathode bezogene Dichte von mindestens 10⁶ Nanostäbchen pro cm² gegeben ist. Zur Herstellung der Nanostäbchen enthaltenden Kathode ist besonders ein Siebdruckverfahren geeignet.

Der Röntgenstrahler des C-Bogen-Röntgengerätes kann dazu vorgesehen sein, verschiedene Röntgenaufnahmen, welche sich hinsichtlich der Dosis voneinander unterscheiden, zu generieren, das heißt mit einer Dosis-Modulation zu arbeiten. Allgemein sind Röntgenstrahlen verschiedener Wellenlängen, wie sie für Multi-Energy- oder Dual-Energy-Aufnahmen vorgesehen sind, durch verschiedene Einstellungen der Anodenspannung erzeugbar. Multi-Energy-Aufnahmen kommen insbesondere in der Angiographie in Betracht.

Was die Gestaltung des Röntgenstrahlers des C-Bogen-Röntgengerätes sowie den Betrieb des Röntgenstrahlers betrifft, sind unter anderem sämtliche Bauformen und Verfahren realisierbar, welche in den Dokumenten WO 2018/086737 A1 und WO 2018/141485 A1 beschrieben sind. Bei dem Detektor des C-Bogen-Röntgengerätes handelt es sich beispielsweise um einen Zeilendetektor.

Unabhängig von der Gestaltung des mindestens einen Röntgenstrahlers sind durch das C-Bogen-Röntgengerät in bevorzugter Verfahrensführung aufeinander folgende Röntgenpulse unterschiedlicher Wellenlänge generierbar. Damit sind mit besonders hoher Zuverlässigkeit und gleichzeitig kurzer Aufnahmedauer unterschiedliche Materialien innerhalb des Untersuchungsvolumens voneinander unterscheidbar.

Gemäß einer vorteilhaften Weiterbildung umfasst das C-Bogen-Röntgengerät zusätzlich zu dem mindestens einen Röntgenstrahler, welcher Nanostäbchen, insbesondere Kohlenstoff-nanoröhren, enthält, einen weiteren Röntgenstrahler. Im Fall einer Ringform oder Polygonform des CNT-Röntgenstrahlers beziehungsweise sonstige Nanostäbchen umfassenden Röntgenstrahlers ist der zusätzliche Röntgenstrahler vorzugsweise zentrisch im ring- oder polygonförmigen Röntgenstrahler angeordnet. Im Vergleich zum letztgenannten, Nanostäbchen aufweisenden Röntgenstrahler ermöglicht der zusätzliche Röntgenstrahler in bevorzugter Ausgestaltung die Emission einer höheren Röntgendosis. Insbesondere kann es sich bei dem zusätzlichen Röntgenstrahler um einen Röntgenstrahler mit rotierender Anode handeln. Dem Röntgenstrahler, welcher Nanostäbchen zur Emission von Elektronen aufweist, und dem weiteren Röntgenstrahler ist ein gemeinsamer Detektor zugeordnet. Bei dem zusätzlichen Röntgenstrahler kann es sich grundsätzlich um einen Röntgenstrahler beliebiger Bauart handeln. Insbesondere kann der zusätzlichen Röntgenstrahler ebenso wie der ringförmige beziehungsweise polygonförmige Röntgenstrahler mindestens eine Kathode, die Nanostäbchen, insbesondere Kohlenstoffnanoröhren, enthält, aufweisen. Beispielsweise enthält der zusätzliche Röntgenstrahler drei Elektronenemitter, die jeweils als Flächenemitter mit Nanostäbchen aufgebaut sind. Besonders bei einem Aufbau des Emitters des zusätzlichen Röntgenstrahlers mit Nanostäbchen kann es sich bei der Anode dieses Röntgenstrahlers auch um eine nicht rotierende Anode handeln.

Gemäß einer alternativen Ausgestaltung sind der Röntgenstrahler und der Röntgendetektor gemeinsam um eine in der Bezugsebene liegende Drehachse schwenkbar. Hierbei sind die Drehbewegungen des Röntgenstrahlers und des Röntgendetektors vorzugsweise elektronisch synchronisiert. Es ist somit eine gemeinsame virtuelle Drehachse des Röntgenstrahlers und des Röntgendetektors gegeben. Diese Ausgestaltung ist auch für einen C-Bogen, welcher als Ganzes nicht schwenkbar ist, geeignet und stellt eine mechanisch insgesamt vereinfachte Ausführungsform dar.

Sofern der Röntgenstrahler und der Röntgendetektor um die genannte Drehachse schwenkbar sind, ist das C-Bogen-Röntgengerät gemäß Anspruch 17 betreibbar. Hierbei werden mehrere Röntgenaufnahmen erstellt, welche sich sowohl hinsichtlich der Einstellung des C-Bogens in dessen Tangentialrichtung, als auch hinsichtlich der Winkeleinstellung des Röntgenstrahlers und des Röntgendetektors, was deren gemeinsame Drehachse betrifft, voneinander unterscheiden. Vorzugsweise wird bei der Erstellung eines Sets an Röntgenaufnahmen der C-Bogen in einer ersten Anzahl verschiedener Positionierungen, was seine Verstellung in Tangentialrichtung betrifft, fixiert wird, wobei in jeder dieser Positionierungen Röntgenaufnahmen mit einer zweiten Anzahl verschiedener Winkeleinstellungen von Röntgenstrahler und Röntgendetektor, das heißt Einstellungen um die genannte Drehachse, generiert werden.

Die Schwenkbarkeit von Röntgenstrahler und Detektor um die genannte Drehachse ist unter anderem zur Tomosynthese nutzbar und ermöglicht allgemein die Erstellung von Multi-Ebenen-Aufnahmen. Ansonsten bietet das C-Bogen-Röntgengerät auch bei nicht am C-Bogen verstellbarem Röntgenstrahler aufgrund dessen beschriebener Ausgestaltung die Möglichkeit, im Vergleich zu herkömmlichen Röntgengeräten bei nicht oder nur moderat erhöhtem apparativen Aufwand besonders hochwertige Röntgenaufnahmen zu erstellen.

Nachfolgend werden mehrere Ausführungsbeispiele anhand einer Zeichnung näher erläutert. Hierin zeigen:
- Fig. 1 und 2: ein erstes Ausführungsbeispiel eines C-Bogen-Röntgengeräts in verschiedenen Betriebspositionen,

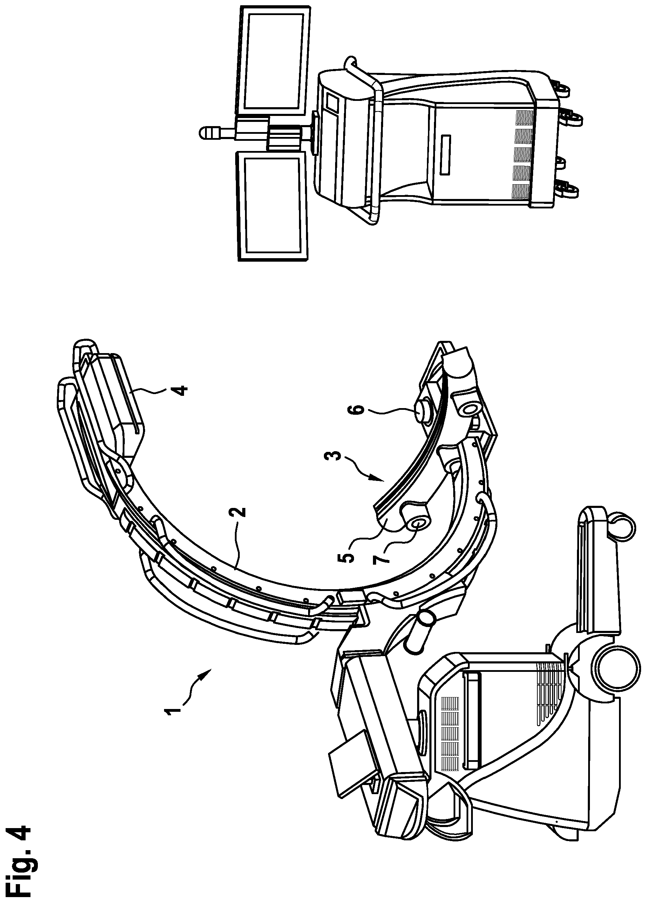

- Fig. 3 und 4: ein zweites Ausführungsbeispiel eines C-Bogen-Röntgengeräts,
- Fig. 5: ein drittes Ausführungsbeispiel eines C-Bogen-Röntgengerätes,
- Fig. 6: ein viertes Ausführungsbeispiel eines C-Bogen-Röntgengerätes,
- Fig. 7: in vereinfachter Darstellung ein Detail des C-Bogen-Röntgengerätes nach Fig. 1 mit veranschaulichtem Strahlengang der aus verschiedenen Röntgenquellen emittierten Röntgenstrahlung,
- Fig. 8: in unterschiedlichen Darstellungen, zumindest in Teilen, einen ringförmigen Röntgenstrahler,
- Fig. 9 bis 11: unterschiedliche Schemata zum Betrieb eines ringförmigen Röntgenstrahlers,
- Fig. 12: einen Röntgenstrahler mit zylindrischer Grundform,
- Fig. 13: einen bogenförmigen Röntgenstrahler,
- Fig. 14: ein Schema zum Betrieb des Röntgenstrahlers nach Fig. 12,
- Fig. 15 bis 20: verschiedene Grundformen weiterer Röntgenstrahler,
- Fig. 21: ein weiteres Ausführungsbeispiel eines C-Bogen-Röntgengeräts in schematischer perspektivischer Ansicht,
- Fig. 22: das C-Bogen-Röntgengerät nach Fig. 22 in schematischer Frontansicht,
- Fig. 23 und 24: um eine gemeinsame virtuelle Achse drehbare Komponenten, nämlich einen Röntgenstrahler beziehungsweise einen Röntgendetektor, des C-Bogen-Röntgengeräts nach Fig. 21,
- Fig. 25: das C-Bogen-Röntgengerät nach Fig. 5 mit im Vergleich zur Einstellung nach Fig. 5 anderer Winkeleinstellung des Röntgenstrahlers um eine virtuelle, zwischen Röntgenstrahler und -detektor verlaufende Drehachse.

Die folgenden Erläuterungen beziehen sich, soweit nicht anders angegeben, auf sämtliche Ausführungsbeispiele.

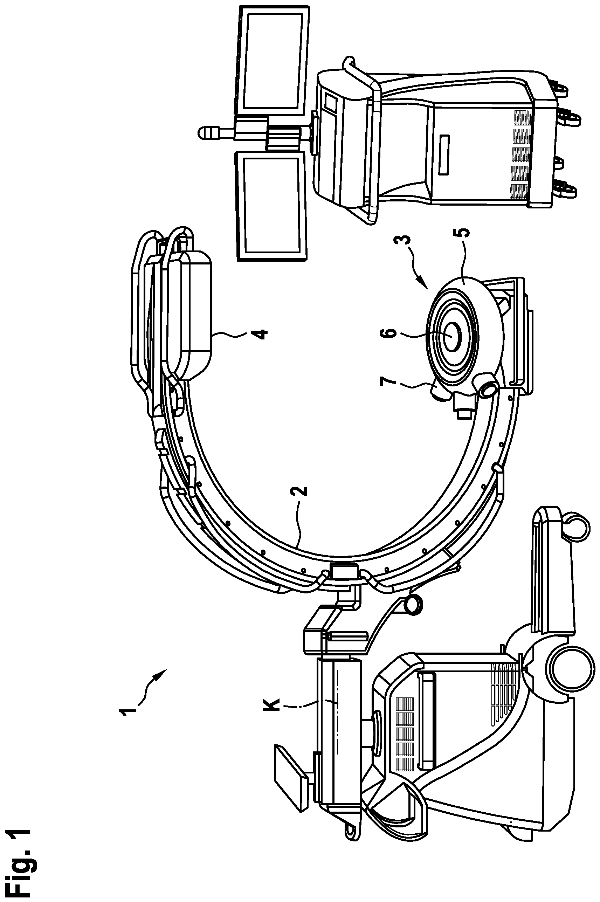

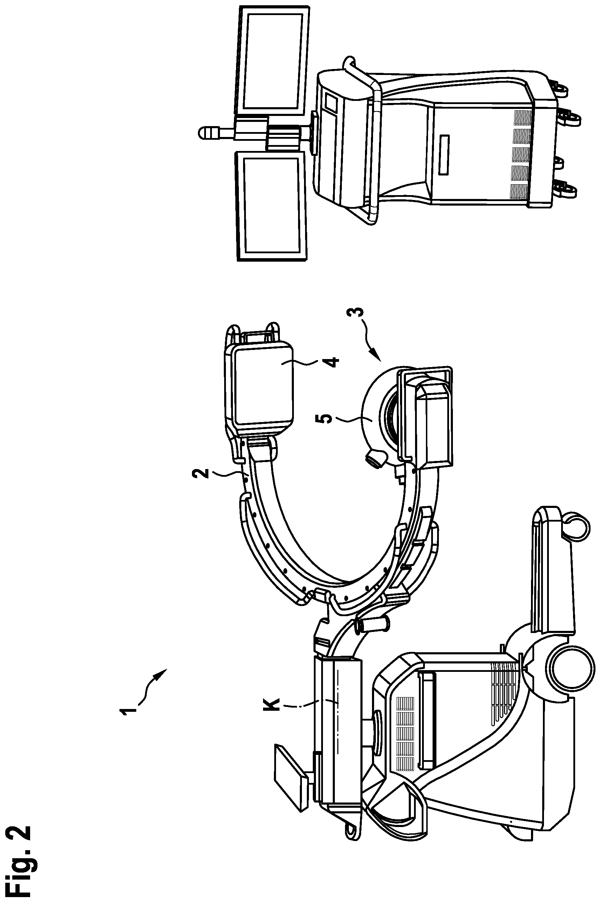

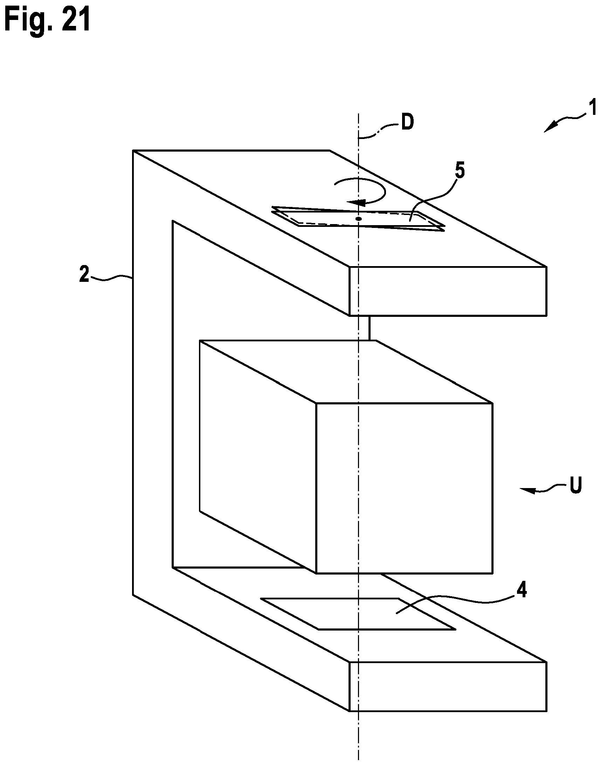

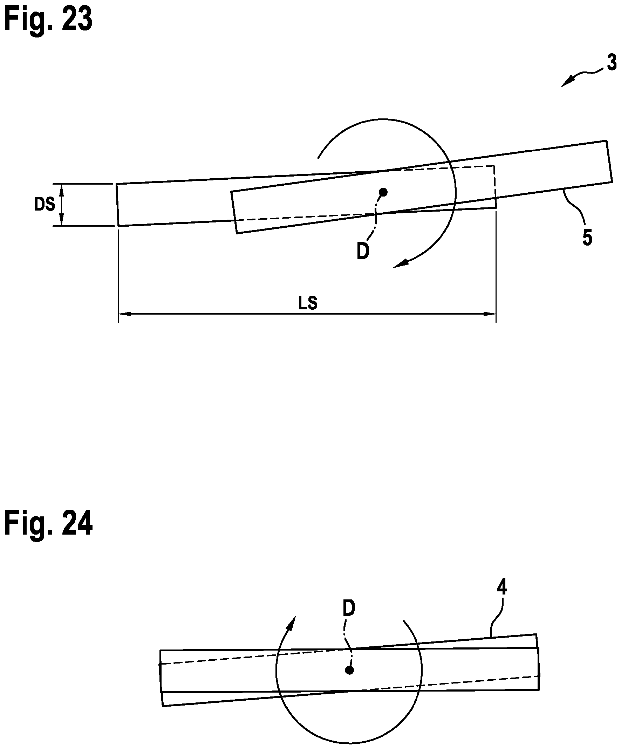

Ein C-Bogen-Röntgengerät 1 weist einen in vielfältiger Weise verstellbaren C-Bogen 2 auf, an welchem eine Röntgenstrahleranordnung 3 und ein zugehöriger Röntgendetektor 4 befestigt sind. Die Verstellbarkeit des C-Bogens 2 gilt nicht für das Ausführungsbeispiel nach den Figuren 21 bis 24. In allen Ausführungsbeispielen definiert der C-Bogen 2 eine Bezugsebene, welche beispielsweise in der Anordnung nach Fig. 1 vertikal und in der Anordnung nach Fig. 2 horizontal ausgerichtet ist.

Die Röntgenstrahleranordnung 3 umfasst in allen Ausführungsbeispielen einen Röntgenstrahler 5 ersten Typs, welcher Feldemissionskathoden mit Kohlenstoffnanoröhren (CNT) umfasst. Der Röntgenstrahler 5 weist eine längliche, optional zu einem ringförmig geschlossenen oder annähernd geschlossenen Gebilde gebogene Form auf. Hierbei kann die geschlossene Form auch durch mehrere Röntgenstrahler 5 gebildet sein. In keinem Fall ist die durch mindestens einen Röntgenstrahler 5 gebildete Ringform oder sonstige ring- oder rahmenförmig geschlossene Form flächig ausgefüllt, etwa in der Form eines Quadrats.

An einen mindestens eine Längs- oder Tangentialrichtung aufweisenden, in sich entweder geraden oder gekrümmten Abschnitt des Röntgenstrahlers 5 kann eine Tangente gelegt werden, welche normal zu der durch den C-Bogen 2 aufgespannten Bezugsebene ausgerichtet ist. Sofern der Röntgenstrahler 5 keine gerade, stabförmige Form aufweist, ist durch diesen eine Ebene aufgespannt, welche orthogonal zur Bezugsebene ausgerichtet ist.

Zusätzlich zum Röntgenstrahler 5 ersten Typs umfasst die Röntgenstrahleranordnung 3 in den Ausgestaltungen nach den Fig. 1, 3 und 6 einen weiteren Röntgenstrahler 6. Dieser zusätzliche Röntgenstrahler 6 arbeitet mit einer zum Zweck der Kühlung rotierenden, nicht dargestellten Anode und ist von daher für die Generierung besonders hoher Röntgendosen geeignet. Der zusätzliche Röntgenstrahler 6 weist ebenso wie der ringförmige Röntgenstrahler 5 Nanostäbchen zur Emission von Elektronen auf. Im Ausführungsbeispiel sind in dem zusätzlichen Röntgenstrahler 6 drei flächige Elektronenemitter, welche jeweils Nanostäbchen aufweisen, angeordnet.

In der Ausgestaltung nach Fig. 1 befindet sich der zusätzliche Röntgenstrahler 6 zentrisch in dem scheibenförmigen, vom ersten Röntgenstrahler 5 umgebenen Raum. Die Mittelachse des zusätzlichen Röntgenstrahlers 6 fällt mit der Symmetrieachse des ringförmigen Röntgenstrahlers 5 zusammen. Die Röntgenstrahler 5, 6 können unabhängig voneinander angesteuert werden.

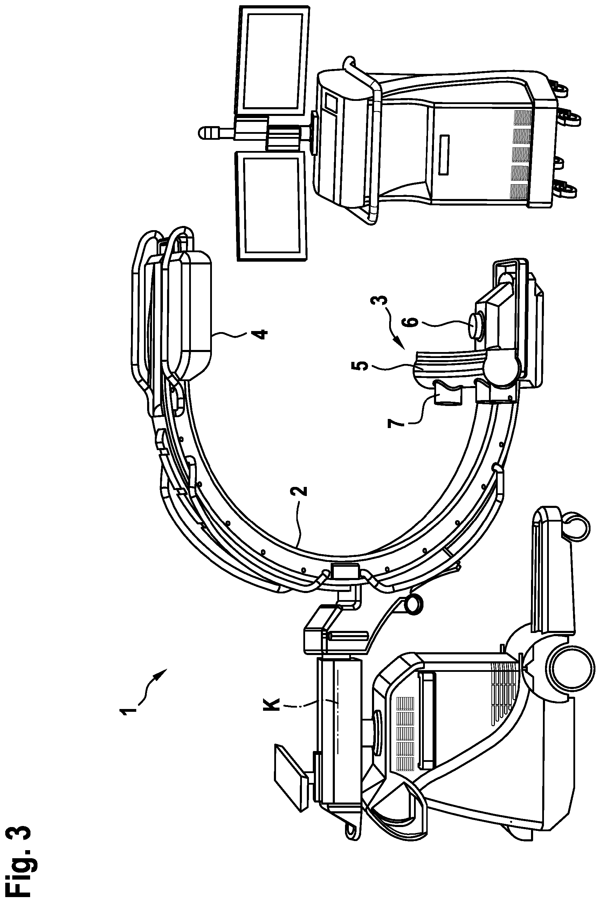

In der Ausgestaltung nach Fig. 3 weist der Röntgenstrahler 5 ersten Typs eine langgestreckte, gebogene Form auf. Insgesamt ist der Röntgenstrahler 5 quer zu der durch den C-Bogen 2 definierten Bezugsebene ausgerichtet. Durch den bogenförmigen Röntgenstrahler 5 ist eine Ebene aufgespannt, welche normal zu der mit K bezeichneten Kippachse des C-Bogens 2 ausgerichtet ist. Die Kippachse K liegt in der Bezugsebene. Der zusätzliche Röntgenstrahler 6 ist im Fall von Fig. 3 neben dem Röntgenstrahler 5 erstens Typs am C-Bogen 2 befestigt.

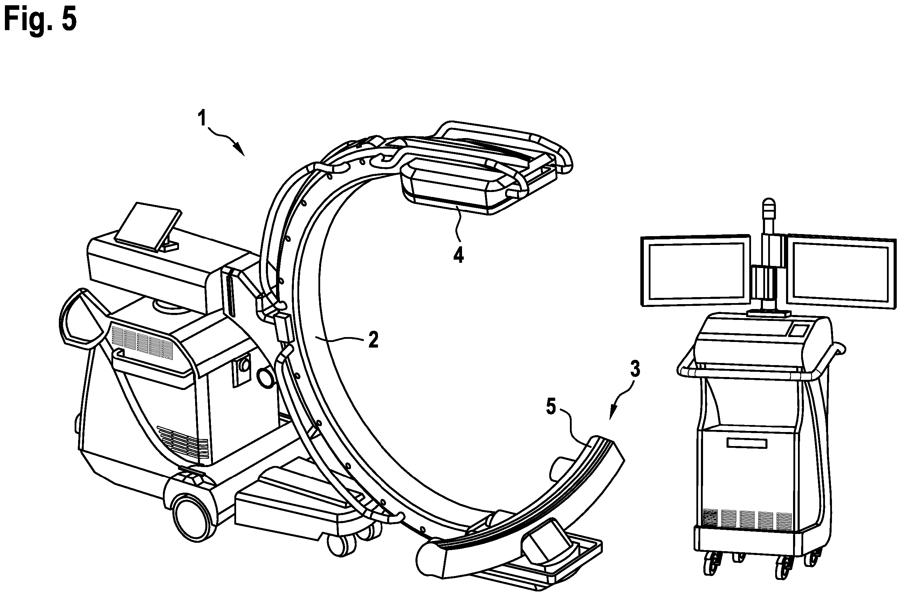

Die Ausführungsform nach Fig. 5 unterscheidet sich von der Ausführungsform nach Fig. 3 durch den Entfall des zusätzlichen Röntgenstrahlers 6. Ebenso könnte ein solcher zusätzlicher Röntgenstrahler 6 bei der Ausführungsform nach Fig. 1 entfallen.

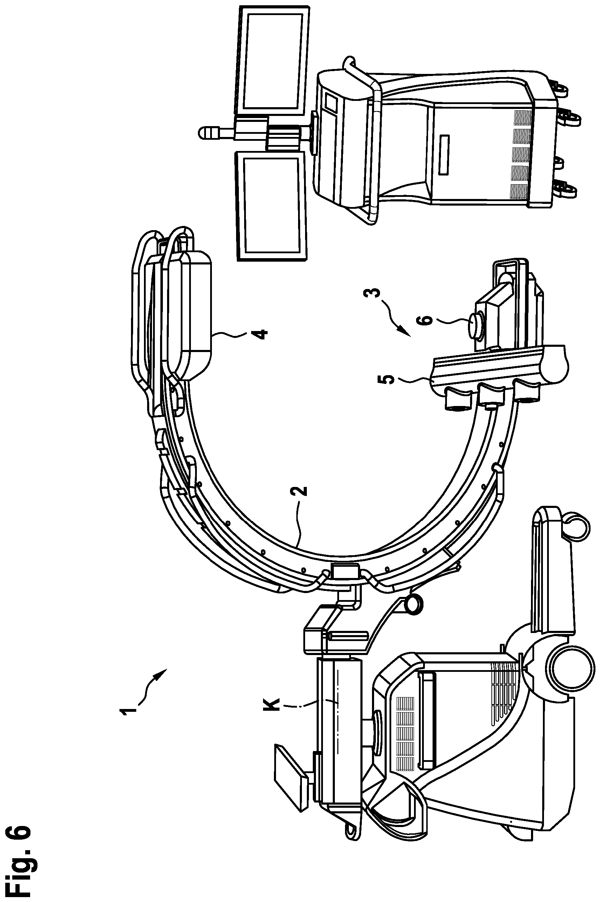

Die Ausführungsform nach Fig. 6 ähnelt der Ausführungsform nach Fig. 3, jedoch hat der Röntgenstrahler 5 in diesem Fall eine gerade, stabförmige Gestalt. Die Längsachse des stabförmigen Röntgenstrahlers 5 ist mit einer Flächennormalen der durch den C-Bogen 2 gebildeten Bezugsebene identisch. Ein zusätzlicher, optionaler Röntgenstrahler 6 ist in der Anordnung nach Fig. 6, ebenso wie in der Anordnung nach Fig. 3, neben dem ersten Röntgenstrahler 5 an einem Ende des C-Bogens 2 befestigt.

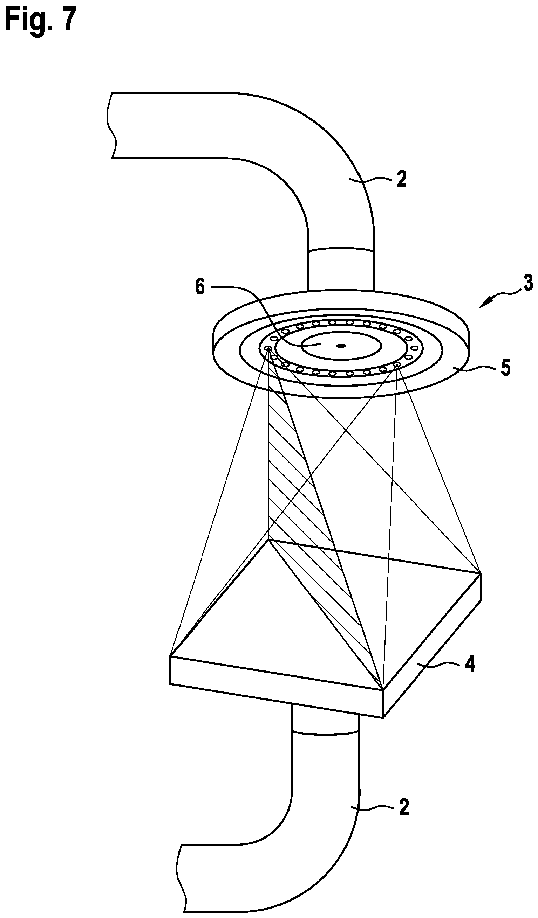

In Fig. 7 ist der Strahlengang von Röntgenstrahlung, welche vom Röntgenstrahler 5 ausgeht, veranschaulicht. Hierbei wird von zwei punktförmigen Quellen der Röntgenstrahlung ausgegangen. Von jeder näherungsweise punktförmigen Quelle breitet sich die Röntgenstrahlung derart aus, dass sie den gesamten, flächigen Detektor 4 trifft. Kollimatorvorrichtungen, die den Strahlengang der Röntgenstrahlung begrenzen, sind in Fig. 7 nicht dargestellt. Aus einer Vielzahl einzelner, jeweils mit einer einzigen Röntgenquelle gewonnener Projektionsbilder sind Tomographiebilder eines Untersuchungsobjektes generierbar, ohne die örtliche Relation zwischen dem C-Bogen 2 und dem Untersuchungsobjekt zu verändern. Durch den Röntgenstrahler 5 nach Fig. 7 sind insgesamt 96 Röntgenquellen exakt definierter Lage gebildet.

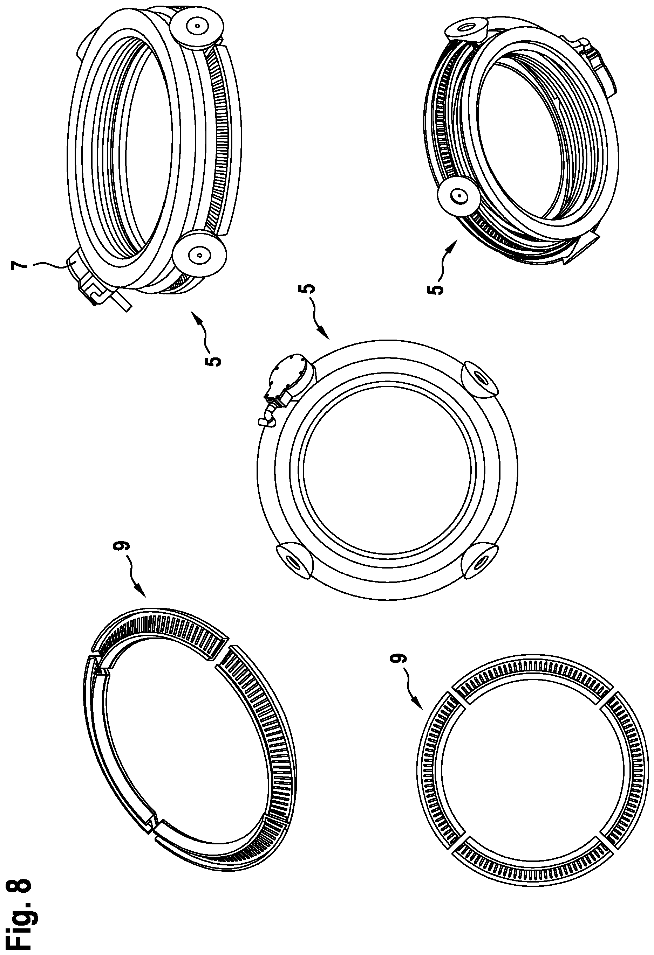

Die in Fig. 8 dargestellte, für das C-Bogen-Röntgengerät 1 nach Fig. 1 geeignete Röntgenstrahleranordnung 3 ist bei Bedarf mit einem hier nicht dargestellten zusätzlichen Röntgenstrahler 6 kombinierbar. Vakuum-Durchführungen sind mit 7, Emitteranordnungen des Röntgenstrahlers 5 mit 9 bezeichnet.

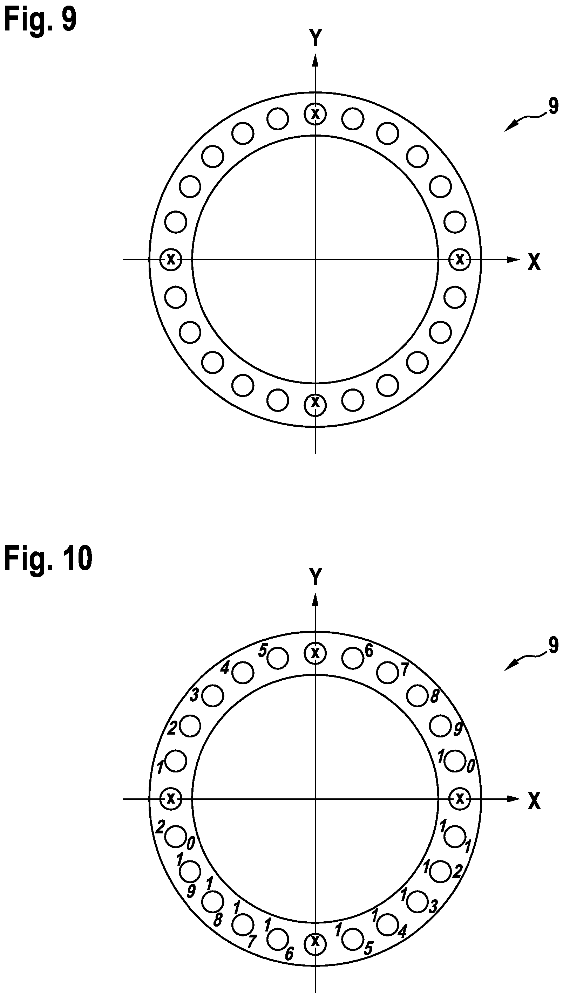

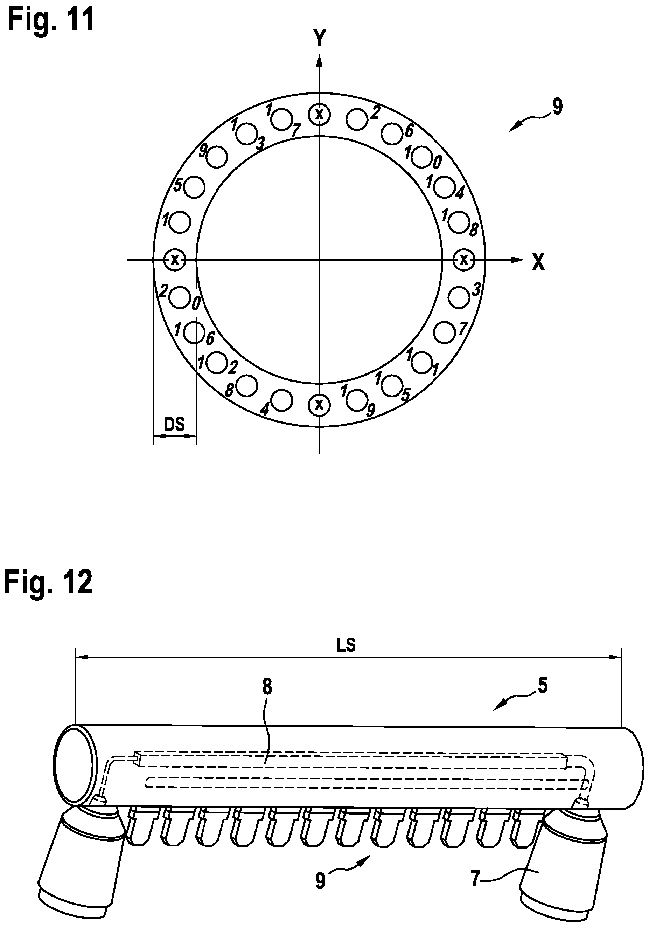

Die Fig. 9 bis 11 veranschaulichen einen möglichen Betriebsmodus der ringförmigen Röntgenstrahleranordnung 3. Sämtliche Elektronenemitter der Röntgenstrahleranordnung 3 sind durch Feldemissionskathoden mit Kohlenstoffnanoröhren (CNT) gebildet. Im ersten Schritt werden beispielsweise, wie in Fig. 9 markiert, vier Emitter angesteuert, welche jeweils im 90° Winkel voneinander beabstandet sind, das heißt auf der X-Achse oder auf der Y-Achse liegen. In den nächsten Schritten können weitere Projektionsaufnahmen beispielsweise entsprechend Fig. 10 oder entsprechend Fig. 11 generiert werden. Im Fall von Fig. 10 werden, ausgehend von demjenigen Emitter, der auf der X-Achse zwischen dem zweiten und dem dritten Quadranten liegt, weitere Emitter in kreisförmig umlaufender Reihenfolge sequentiell angesteuert.

Dagegen werden im Fall von Fig. 11 nacheinander Emitter, die in verschiedenen Quadranten liegen, aktiviert, so dass nacheinander stets zwei Emitter angesteuert werden, zwischen welchen im Vergleich zum Betriebsmodus nach Fig. 10 ein großer Abstand in Umfangsrichtung des Röntgenstrahlers 5 gegeben ist. Der in Fig. 11 veranschaulichte Betriebsmodus hat den Vorteil, dass bereits basierend auf Projektionsbildern, die nur mit einem Teil der vorhandenen Emitter generiert wurden, dreidimensionale Bilder akzeptabler Qualität gewonnen werden können.

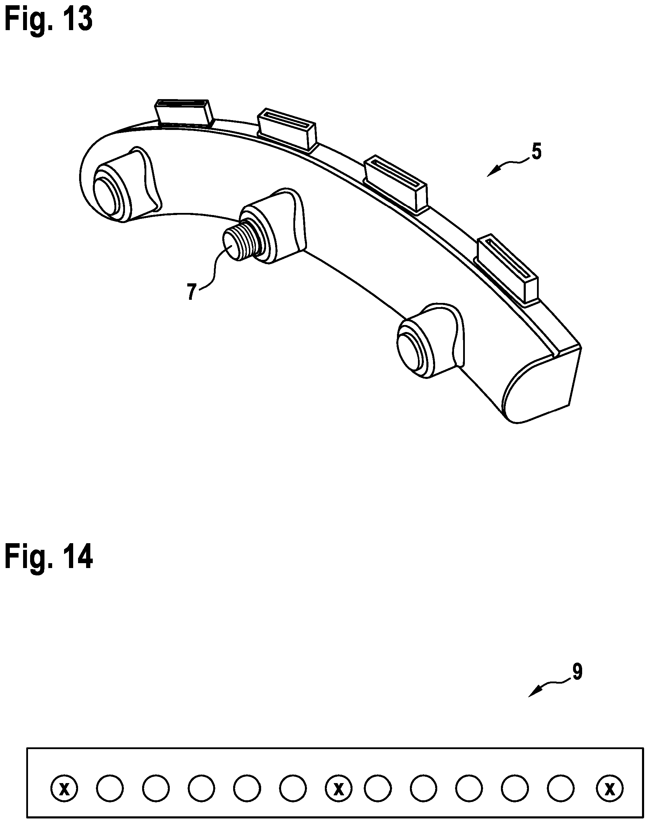

Die Fig. 12 und 13 zeigen in perspektivischer, teilweise transparenter (Fig. 12) Darstellung die Röntgenstrahler 5 nach Fig. 6 beziehungsweise nach Fig. 3. Die in Fig. 12 mit 8 bezeichnete Anode des Röntgenstrahlers 5 ist durch ein elektrisch nicht leitendes Öl, welches die Anode 8 durchströmt, gekühlt. Durch jeden der Röntgenstrahler 5 ist eine Längsrichtung definiert, welche im Fall von Fig. 13 durch die Tangentialrichtung des durch den Röntgenstrahler 5 beschriebenen Bogens definiert ist, wobei die Tangente mittig zwischen den beiden Enden des Bogens an diesen anzulegen ist. In der Ausgestaltung nach Fig. 12 bezeichnet LS die gesamt Länge des Röntgenstrahlers 5. Die Gesamtlänge LS ist stets in Längsrichtung einer durch den Röntgenstrahler 5 beschriebenen langgestreckten Struktur, im Fall von Fig. 12 eines Zylinders mit kreisförmigem Querschnitt, zu messen.

Die Fig. 14 dient der Veranschaulichung eines möglichen Betriebsmodus des Röntgenstrahlers 5 nach Fig. 12. In diesem Fall werden zunächst drei CNT-Kathoden des Röntgenstrahlers 5 angesteuert, welche sich in der Mitte beziehungsweise an den beiden Enden des Röntgenstrahlers 5 befinden. Was die folgende Ansteuerung der restlichen Elektronenemitter betrifft, existieren verschiedene Möglichkeiten: Beispielsweise können die restlichen Elektronenemitter linear durchlaufend aktiviert werden, was prinzipiell dem Betriebsmodus nach Fig. 10 entspricht. Ebenso ist es möglich, nacheinander einzelne Emitter anzusteuern, welche weiter voneinander beabstandet sind, wie grundsätzlich bereits anhand Fig. 11 erläutert wurde. Auch eine Aktivierung einzelner Elektronenemitter nach dem Zufallsprinzip (random) ist möglich.

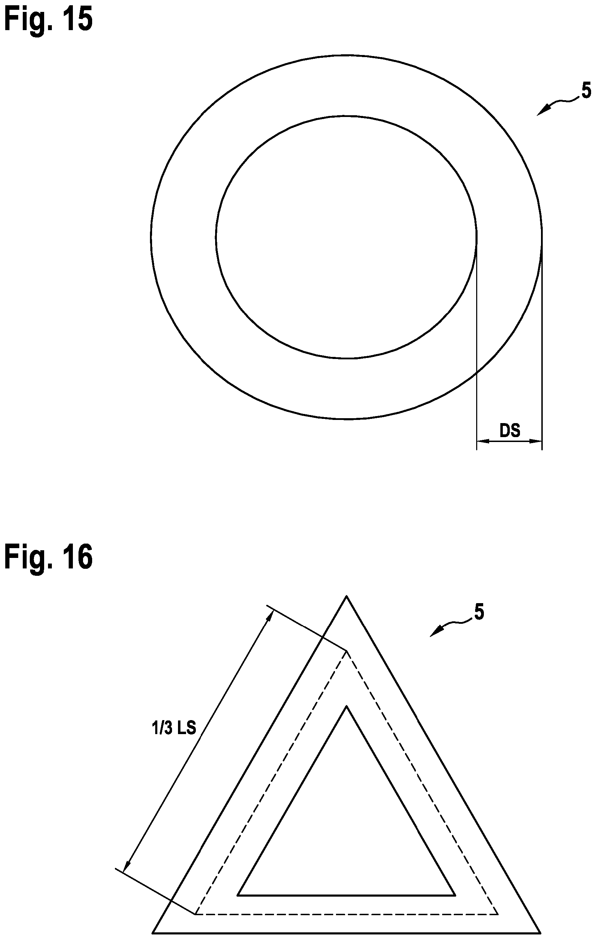

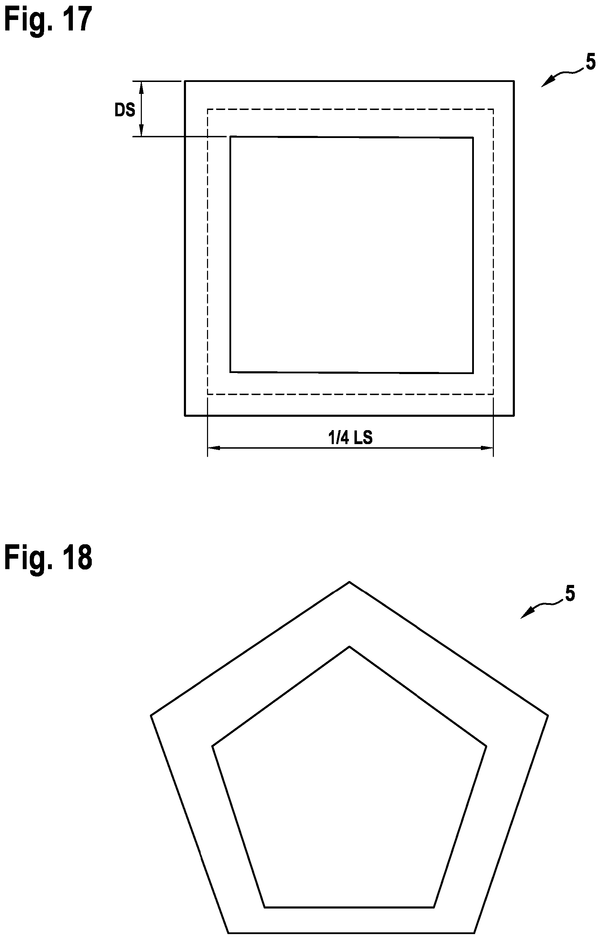

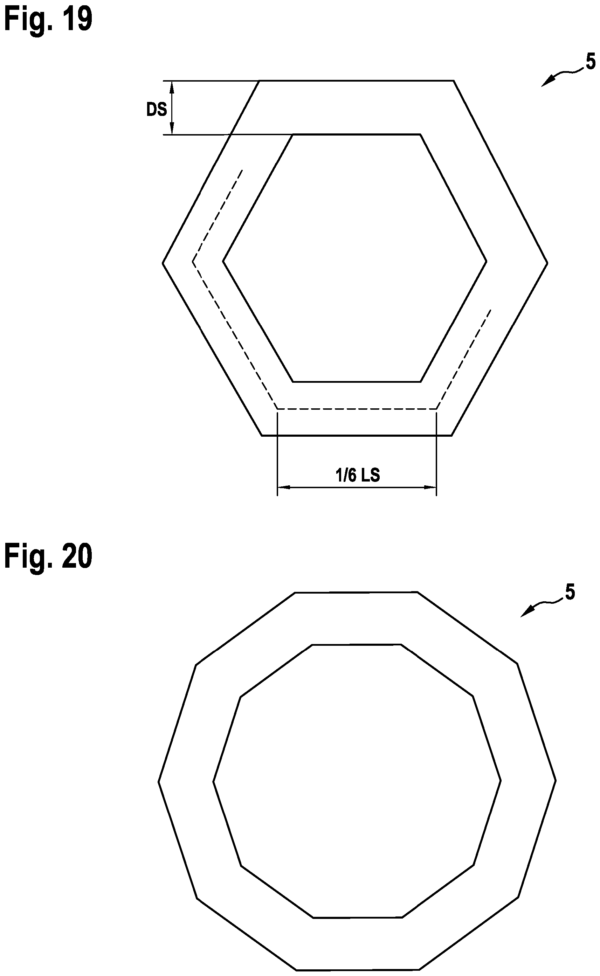

In den Figuren 15 bis 20 sind zusätzlich zum ringförmigen Röntgenstrahler 5 nach Fig. 1 weitere mögliche Formen von Röntgenstrahlern 5, welche für das C-Bogengerät 1 verwendbar sind, skizziert. Hierbei handelt es sich um eine dreieckige, quadratische, fünfeckige, sechseckige und zehneckige Form. Auch weitere geschlossene Formen, beispielsweise eine Oktagonform, sind möglich.

Mit DS ist der Durchmesser des Querschnitts der langgestreckten Struktur des Röntgenstrahlers 6 bezeichnet. Im Fall von Fig. 15 handelt es sich bei der langgestreckten Struktur um einen Torus. Im Fall eines nicht kreisförmigen Querschnitts der langgestreckten Struktur, wie in den Fällen nach den Figuren 16 bis 20, ist DS als maximaler Durchmesser des Querschnitts der langgestreckten Struktur definiert. Die teilweise gestrichelt eingezeichnete Linie, längs welcher die gesamte Erstreckung LS zu messen ist, ist im Fall eines polygonförmigen Röntgenstrahlers 5 entsprechend der betreffenden Polygonform mehrfach abgeknickt. In allen Fällen beträgt die Erstreckung LS mehr als das Vierfache des Durchmessers DS.

In den in den Figuren 15 bis 20 skizzierten Fällen befindet sich der optionale, zusätzliche Röntgenstrahler 6 vorzugsweise, ebenso wie in der Anordnung nach Fig. 1, in der Mitte der jeweiligen Form. Damit schneidet die durch den C-Bogen 2 definierte Bezugsebene den zusätzlichen Röntgenstrahler 6, während der mit CNT-Emittern arbeitende Röntgenstrahler 5 beidseitig aus dieser Bezugsebene auskragt.

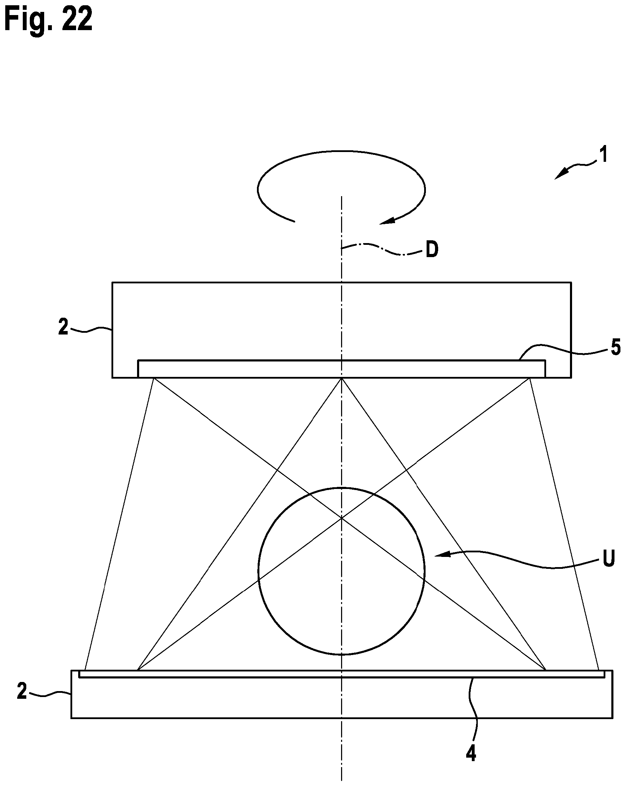

Im Ausführungsbeispiel nach den Figuren 21 bis 24 ist der C-Bogen 2 während einer röntgentechnischen Untersuchung fest im Raum angeordnet. Lediglich der Röntgenstrahler 5 und der Röntgendetektor 4 sind um eine gemeinsame, virtuelle Drehachse D schwenkbar. Die Synchronisierung der Schwenkbewegungen des Röntgenstrahlers 5 und des Röntgendetektors 4 erfolgt elektronisch. Wie aus Fig. 21 hervorgeht, weisen der Röntgenstrahler 5 sowie der Röntgendetektor 4 jeweils eine langgestreckte Grundform auf. In der Anordnung nach Fig. 21 ist der Röntgenstrahler 5 ebenso wie der Röntgendetektor 4 quer zur Bezugsebene, welche durch den C-Bogen 2 aufgespannt ist, ausgerichtet. In der Ansicht nach Fig. 22 ist die Bezugsebene orthogonal zur Zeichenebene ausgerichtet, wobei die Drehachse D in der Bezugsebene liegt. Ein zwischen dem Röntgenstrahler 5 und dem Röntgendetektor 4 liegender Untersuchungsbereich U wird von der Drehachse D geschnitten.

Der Röntgenstrahler 5 des Röntgengerätes 1 nach Fig. 21 ist zur Emission fächerförmiger Röntgenstrahlenbündel geeignet, wobei die Ebene, in welcher der Fächer liegt, während einer Röntgenaufnahme durch diskontinuierliche oder kontinuierliche Schwenkung des Röntgenstrahlers 5 um die Drehachse D in vielfältiger Weise verstellbar ist. Die geringen Massen und Trägheitsmomente des Röntgenstrahlers 5 und des Röntgendetektors 4 im Vergleich zum gesamten C-Bogen 2 ermöglichen in kurzer Taktung aufeinander folgende, bei verschiedenen Winkeleinstellungen des Röntgenstrahlers 5 und des Röntgendetektors 4 vorzunehmende Röntgenaufnahmen, wobei bei jeder Röntgenaufnahme die aus dem Röntgenstrahler 5 und dem Röntgendetektors 4 gebildete Baugruppe im Stillstand sein kann. Ein damit generiertes Set an Projektionen ermöglicht mit im Vergleich zu herkömmlichen Anlagen geringem apparativen Aufwand sowie Zeitaufwand die Bereitstellung von Schnittbildern und Volumendaten. Als Röntgendetektor 4 ist im Ausführungsbeispiel nach Fig. 21 ein Zeilendetektor vorgesehen.

Der Röntgenstrahler 5 ist ebenso wie der Röntgendetektor 4 in einem Winkelbereich von mindestens +20° bis -20°, was die Verstellung um die Drehachse D betrifft, verstellbar. Die Verstellung kann beispielsweise in 40 Stufen erfolgen. Sofern zusätzlich eine - in Fig. 21 nicht erkennbare - Verstellung in Tangentialrichtung des C-Bogens 2 vorgesehen ist, das heißt um eine gedachte Querachse, die normal zur Bezugsebene, also in Längsrichtung von Röntgenstrahler 5 und Röntgendetektor 4, sofern sich diese in ihrer Mittelstellung befinden, ausgerichtet ist, ergibt sich insgesamt eine Vielzahl möglicher Einstellungen. Ist beispielsweise eine Verstellung des C-Bogens um die Querachse in 60 Stufen vorgesehen, ergeben sich insgesamt 40 x 60 = 2.400 mögliche Einstellungen der aus Röntgenstrahler 5 und Röntgendetektor 4 gebildeten Baugruppe.

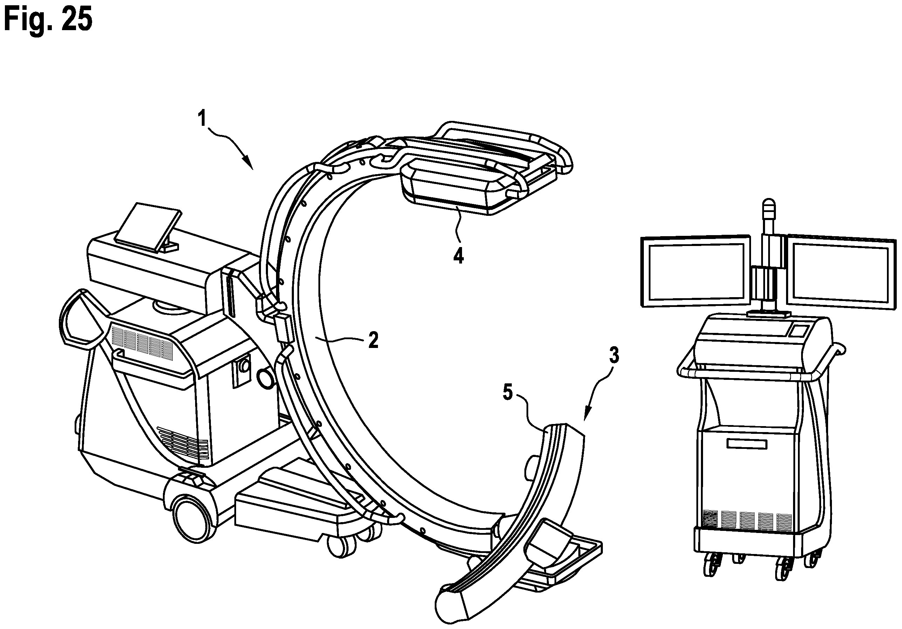

Die Verstellung des Röntgenstrahlers 5 um die Drehachse D, welche mittig den Röntgenstrahler 5 sowie den Röntgendetektor 4 schneidet, ist auch in Fig. 25, welche das Ausführungsbeispiel nach Fig. 5 zeigt, illustriert. Auch in allen anderen Ausführungsformen des C-Bogen-Röntgengerätes 1 mit einem langgestreckten, geraden oder gebogenen Röntgenstrahler 5 kann dessen Verstellbarkeit um die Drehachse 5 vorgesehen sein.

### Bezugszeichenliste

- 1: C-Bogen-Röntgengerät
- 2: C-Bogen
- 3: Röntgenstrahleranordnung
- 4: Röntgendetektor
- 5: Röntgenstrahler ersten Typs (mit Nanostäbchen)
- 6: weiterer Röntgenstrahler
- 7: Vakuum-Durchführung
- 8: Anode
- 9: Emitteranordnung

- DS: Durchmesser des Querschnitts der langgestreckten Struktur des Röntgenstrahlers
- K: Kippachse
- LS: Erstreckung in Längsrichtung der langgestreckten Struktur des Röntgenstrahlers
- U: Untersuchungsbereich

## Patentansprüche

1. C-Bogen-Röntgengerät, mit einem Röntgenstrahler (5) und einem Röntgendetektor (4), welche an einem in einer Bezugsebene liegenden C-Bogen (2) gehalten sind, wobei der Röntgenstrahler (5) Nanostäbchen als Elektronenemitter umfasst und eine langgestreckte, schlauchförmige Struktur beschreibt, **dadurch gekennzeichnet, dass** der Röntgenstrahler (5) eine gekrümmte, langgestreckte Form aufweist, wobei eine mittig an den Röntgenstrahler gelegte Tangente eine Flächennormale der Bezugsebene darstellt und die gekrümmte Form des Röntgenstrahlers eine orthogonal zur Bezugsebene ausgerichtete Ebene aufspannt, und wobei die gesamte Erstreckung des Röntgenstrahlers (5) mindestens das Vierfache des quer zur langgestreckten Struktur gemessenen Durchmessers des Querschnitts des Röntgenstrahlers (5) beträgt.

2. Röntgengerät nach Anspruch 1, **dadurch gekennzeichnet, dass** Kohlenstoffnanoröhren (CNT) als Nanostäbchen zur Emission von Elektronen vorgesehen sind.

3. Röntgengerät nach Anspruch 2, **dadurch gekennzeichnet, dass** zumindest ein Teil der Nanostäbchen als ein- oder mehrwandige Kohlenstoffnanoröhren oder ein- oder mehrwandige Hetero-Stickstoff-Kohlenstoffnanoröhren ausgebildet ist.

4. Röntgengerät nach Anspruch 2 oder 3, **dadurch gekennzeichnet, dass** zumindest ein Teil der Nanostäbchen Boride der seltenen Erden, Metalloxide, Metallsulfide, Nitride, Carbide oder Silicium enthält.

5. Röntgengerät nach einem der Ansprüche 2 bis 4, **dadurch gekennzeichnet, dass** die Nanostäbchen eine Länge von weniger als 20 µm und einen Durchmesser von weniger als 10 nm aufweisen, wobei eine auf die Fläche der zur Emission von Elektronen ausgebildeten Kathode bezogene Dichte von mindestens 10⁶ Nanostäbchen pro cm² gegeben ist.

6. Röntgengerät nach einem der Ansprüche 1 bis 5, **dadurch gekennzeichnet, dass** es zusätzlich zum Nanostäbchen, insbesondere Kohlenstoffnanoröhren (CNT), enthaltenden Röntgenstrahler (5) einen weiteren Röntgenstrahler (6) umfasst.

7. Röntgengerät nach Anspruch 6, **dadurch gekennzeichnet, dass** der weitere Röntgenstrahler (6) eine rotierende Anode aufweist.

8. Röntgengerät nach einem der Ansprüche 1 bis 7, **dadurch gekennzeichnet, dass** der Röntgenstrahler (5) und der Röntgendetektor (4) gemeinsam um eine in der Bezugsebene liegende Drehachse (D) schwenkbar sind.

9. Röntgengerät nach Anspruch 8, **dadurch gekennzeichnet, dass** der Röntgenstrahler (5) zur Emission fächerförmiger Röntgenstrahlenbündel ausgebildet ist.

10. Röntgengerät nach Anspruch 8 oder 9, **dadurch gekennzeichnet, dass** der Röntgenstrahler (5) und der Röntgendetektor (4), was Drehungen um die Drehachse (D) betrifft, elektronisch synchronisiert sind.

11. Röntgengerät nach einem der Ansprüche 8 bis 10, **dadurch gekennzeichnet, dass** der C-Bogen (2) in dessen Tangentialrichtung, das heißt um eine gedachte, quer zur Bezugsebene liegende Schwenkachse, verstellbar ist.

12. Verfahren zum Betrieb eines Röntgengerätes nach Anspruch 11, wobei mehrere Röntgenaufnahmen erstellt werden, welche sich sowohl hinsichtlich der Einstellung des C-Bogens (2) in dessen Tangentialrichtung, als auch hinsichtlich der Winkeleinstellung des Röntgenstrahlers (5) und des Röntgendetektors (4), was deren gemeinsame Drehachse (D) betrifft, voneinander unterscheiden.

13. Verfahren nach Anspruch 12, **dadurch gekennzeichnet, dass** bei der Erstellung eines Sets an Röntgenaufnahmen der C-Bogen (2) in einer ersten Anzahl verschiedener Positionierungen, was seine Verstellung in Tangentialrichtung betrifft, fixiert wird und in jeder dieser Positionierungen Röntgenaufnahmen mit einer zweiten Anzahl verschiedener Winkeleinstellungen von Röntgenstrahler (5) und Röntgendetektor (4) generiert werden.

## Claims

1. A C-arm X-ray apparatus comprising an X-ray emitter (5) and an X-ray detector (4) which are held on a C-arm (2) that extends in a reference plane, wherein the X-ray emitter (5) comprises nanorods as electron emitters and defines an elongated, hose-like structure, **characterized in that** the X-ray emitter (5) has a curved, elongated shape , wherein a tangent orientated on the centre of the X-ray emitter represents a surface normal of the reference plane, and the curved shape of the X-ray emitter spans a plane aligned orthogonally to the reference plane, and wherein the total extension of the X-ray emitter (5) is at least four times greater than the diameter of the cross section of the X-ray emitter (5) measured transversely to the elongated structure.

2. An X-ray apparatus according to Claim 1, **characterized in that** carbon nanotubes (CNT) are provided as nanorods for the emission of electrons.

3. The X-ray apparatus according to Claim 2, **characterized in that** at least some of the nanorods have the form of single- or multi-wall carbon nanotubes or single- or multi-wall heteronitrogen carbon nanotubes.

4. The X-ray apparatus according to Claim 2 or 3, **characterized in that** at least some of the nanorods contain borides of rare earth metals, metal oxides, metal sulphides, nitrides, carbides, or silicon.

5. The X-ray apparatus according to any one of Claims 2 to 4, **characterized in that** the nanorods have a length of less than 20 µm and a diameter of less than 10 nm, wherein a density of at least 10⁶ nanorods per cm² related to the area of the cathode designed for emitting the electrons is given.

6. The X-ray apparatus according to any one of Claims 1 to 5, **characterized in that** besides the X-ray emitter (5) that contains nanorods, in particular carbon nanotubes (CNT), it also comprises a further X-ray emitter (6).

7. The X-ray apparatus according to Claim 6, **characterized in that** the further X-ray emitter (6) has a rotating anode.

8. The X-ray apparatus according to any one of Claims 1 to 7, **characterized in that** the X-ray emitter (5) and the X-ray detector (4) can be swivelled together about an axis of rotation (D) aligned in the reference plane.

9. The X-ray apparatus according to Claim 8, **characterized in that** the X-ray emitter (5) is designed to emit fan-shaped X-ray beam bundles.

10. The X-ray apparatus according to Claim 8 or 9, **characterized in that** the X-ray emitter (5) and the X-ray detector (4) are synchronised electronically regarding revolutions about the axis of rotation (D).

11. The X-ray apparatus according to any one of Claims 8 to 10, **characterized in that** the C-arm (2) is displaceable in the tangential direction thereof, that is to say about a notional swivel axis aligned transversely to the reference plane.

12. A method for operating an X-ray apparatus according to Claim 11, wherein multiple X-ray images are created that differ from each other both in view of the adjustment of the C-arm (2) in the tangential direction thereof, and in view of the angular adjustment of the X-ray emitter (5) and the X-ray detector (4) with respect to their common axis of rotation (D).

13. The method according to Claim 12, **characterized in that** during creation of a set of X-ray images the C-arm (2) is fixed in a first number of different positions with regard to its displacement in the tangential direction, and in each of these positions X-ray images are generated with a second number of different angular settings between X-ray emitter (5) and X-ray detector (4).

## Revendications

1. Appareil de radiographie à bras en C, comprenant un émetteur de rayons (5) et un détecteur de rayons (4) qui sont maintenus sur un bras en C (2) reposant dans un plan de référence, dans lequel l'émetteur de rayons (5) comprend des nanotiges en tant qu'émetteurs d'électrons et décrit une structure étirée en longueur et en forme de tuyau, **caractérisé en ce que** l'émetteur de rayons (5) présente une forme courbe étirée en longueur, dans lequel une tangente placée au centre de l'émetteur de rayons représente une normale de surface du plan de référence, et la forme courbe de l'émetteur de rayons fixe un plan aligné de manière orthogonale au plan de référence, et dans lequel l'allongement total de l'émetteur de rayons (5) fait au moins quatre fois le diamètre mesuré transversalement à la structure étirée en longueur de la section transversale de l'émetteur de rayons (5).

2. Appareil de radiographie selon la revendication 1, **caractérisé en ce que** des nanotubes de carbone (CNT) sont prévus en tant que nanotiges pour émettre des électrons.

3. Appareil de radiographie selon la revendication 2, **caractérisé en ce qu'**au moins une partie des nanotiges est conçue en tant que nanotubes de carbone à une seule paroi ou plus ou hétéro-nanotubes carbone-azote à une seule paroi ou plus.

4. Appareil de radiographie selon la revendication 2 ou 3, **caractérisé en ce qu'**au moins une partie des nanotiges contient du borure des terres rares, de l'oxyde métallique, du sulfure métallique, du nitrure, du carbure ou du silicium.

5. Appareil de radiographie selon l'une des revendications 2 à 4, **caractérisé en ce que** les nanotiges présentent une longueur de moins de 20 µm et un diamètre de moins de 10 nm, dans lequel une densité d'au moins 10⁶ nanotiges par cm² rapportée à la surface de la cathode formée pour l'émission d'électrons est donnée.

6. Appareil de radiographie selon l'une des revendications 1 à 5, **caractérisé en ce qu'**il y a un émetteur de rayons supplémentaire (6) en plus de l'émetteur de rayons (5) contenant des nanotiges, en particulier des nanotubes de carbone (CNT).

7. Appareil de radiographie selon la revendication 6, **caractérisé en ce que** l'émetteur de rayons supplémentaire (6) présente une anode rotative.

8. Appareil de radiographie selon l'une des revendications 1 à 7, **caractérisé en ce que** l'émetteur de rayons (5) et le détecteur de rayons (4) peuvent basculer conjointement sur un pivot (D) reposant dans le plan de référence.

9. Appareil de radiographie selon la revendication 8, **caractérisé en ce que** l'émetteur de rayons (5) es conçu pour émettre un faisceau de rayons en éventail.

10. Appareil de radiographie selon la revendication 8 ou 9, **caractérisé en ce que** l'émetteur de rayons (5) et le détecteur de rayons (4) sont synchronisés électroniquement, en ce qui concerne les rotations sur le pivot (D).

11. Appareil de radiographie selon l'une des revendications 8 à 10, **caractérisé en ce que** le bras en C (2) est réglable dans sa direction tangentielle, c'est à dire sur un pivot imaginaire transversal au plan de référence.

12. Procédé pour le fonctionnement d'un appareil de radiographie selon la revendication 11, dans lequel plusieurs clichés radiographiques sont produits qui se différencient les uns des autres tant au niveau du réglage du bras en C (2) dans sa direction tangentielle qu'au niveau du réglage d'angle de l'émetteur de rayons (5) et du détecteur de rayons (4) en ce qui concerne leur pivot (D) commun.

13. Procédé selon la revendication 12, **caractérisé en ce que** lors de la production d'un jeu de clichés radiographiques, le bras en C (2) est fixé dans un premier nombre de différents positionnements, en ce qui concerne son déplacement dans la direction tangentielle, et dans chacune de ces positions, des clichés radiographiques sont pris avec un second nombre de différents réglages d'angle de l'émetteur de rayons (5) et du détecteur de rayons (4).
